Europäisches Patentamt

European Patent Office

Office européen des brevets

(11) Publication number: **0 269 279**

**A2**

# EUROPEAN PATENT APPLICATION

(21) Application number: **87309610.1**

(22) Date of filing: **30.10.87**

(51) Int. Cl.⁴: **A61K 35/14** ,
//G01N33/564,G01N33/566,G01-
N33/574

(30) Priority: **21.11.86 US 934272**

(43) Date of publication of application:
**01.06.88 Bulletin 88/22**

(84) Designated Contracting States:
**AT BE CH DE ES FR GB GR IT LI LU NL SE**

(71) Applicant: **IMRE CORPORATION**
**130 Fifth Avenue North**
**Seattle Washington 98109-4933(US)**

(72) Inventor: **Jones, Frank R.**
**9825 - 23rd Street S.W.**
**Edmonds Washington(US)**
Inventor: **Snyder, Harry W.**
**23923 - 101st Place W.**
**Edmonds Washington(US)**
Inventor: **Balint, Joseph P.**
**2442 N.W. Market Street Box 310**
**Seattle Washington(US)**

(74) Representative: **Harrison, David Christopher**
**et al**
**MEWBURN ELLIS & CO 2/3 Cursitor Street**
**London EC4A 1BQ(GB)**

(54) **Extracorporeal removal of immunoglobulin-G and circulating immune complexes.**

(57) A method for removing immunoglobulin-G and immune complexes from blood comprises withdrawing a small, discrete volume of blood from a patient, separating the blood volume into its cellular and plasma components, immediately reinfusing the cellular components to the patient, passing the plasma components through a protein A immunoadsorbent, and reinfusing the treated plasma to the patient. By treating relatively small volumes of blood and employing an inert, non-colloidal protein A immunoadsorbent, typically protein A covalently bound to microcrystalline silica, greatly reduced side effects are observed without loss of efficacy. In treatment of cancer and autoimmune diseases, the treatment protocol is repeated periodically, usually at least once per week for a period of four weeks or longer. A cartridge for use in such treatment is described, in which a charge of the immunoadsorbent may be contained in a cylinder (12).

FIG._I.

## EXTRACORPOREAL REMOVAL OF IMMUNOGLOBULIN-G AND CIRCULATING IMMUNE COMPLEXES

The present invention is related generally to a method for the extracorporeal removal of immunoglobulin-G (IgG) and immune complexes from a patient's plasma. More particularly, it is related to a non-continuous method where discrete volumes of blood are drawn, the plasma separated from the blood, and the entire volume of plasma treated prior to reinfusion into the patient.

The extracorporeal removal of immunoglobulins and circulating immune complexes from a patient's blood may be useful in a variety of circumstances. For example, there is evidence that such removal has therapeutic value in the treatment of various cancers and autoimmune diseases. It is suspected that some cancer patients develop immune complexes consisting of the patient's own antibodies (IgG) and an antigen specific for or associated with the cancer and that such complexes act as "blocking factors" which inhibit the patient's immune response to the cancer. By adsorbing IgG and its complexes from the blood, the patient's natural immune defenses can resume their proper function. Autoimmune disorders involve the production of antibodies which are specific for the patient's own body tissues. Severe harm can arise from such a misdirected immune response, causing illness and even death to the patient. Removal of the antibodies may protect the body from further damage. The removal of IgG and immune complexes from a patient with an autoimmune disease can also result in an immune modulation and therapeutic benefit.

Heretofore, the preferred method for removing IgG and immune complexes from blood has involved extracorporeal immunoperfusion where blood is continuously removed from a patient and separated into its cellular and plasma components. The plasma is directed through an immunoadsorbent, typically a protein A or killed Staphylococcus aureus column, to remove the IgG and its complexes. The treated plasma is then recombined with the cellular components and returned to the patient. Usually, at least one blood volume will be removed during each treatment, and treatments will be repeated periodically.

Although such treatments have shown beneficial results, they suffer from several disadvantages. Continuous plasma perfusion requires expensive equipment and highly trained personnel to treat each patient, and each treatment may last several hours. Thus, the expense and discomfort of the procedure are considerable. Moreover, patients undergoing such treatments have suffered significant side effects, such as nausea, chills, vomiting, fever, and, in the worst cases, hypotension, hypertension, and conjestive heart failure. Additionally, such treatments have not yet proven to be effective to the extent desirable.

For these reasons, it would be desirable to provide alternative methods for extracorporeal plasma perfusion. In particular, it would be desirable that such methods would provide enhanced therapeutic value in treating diseases, particularly cancer and autoimmune diseases, would involve a simpler, less costly and less time consuming treatment protocol, and would subject the patient to less severe side effects.

Columns of killed Staphylococcus aureus Cowan I have been used to treat patients suffering from various forms of cancer. See, e.g., Bansal et al. (1978) Cancer 42:1-18; Ray et al. (1980) Cancer 45:2633-2638; Messerschmidt et al. (1982) Cancer Treat. Rep. 66:2027-2031; and Holohan et al. (1982) Cancer Res. 42:3663-3668. The use of a colloidion charcoal matrix having non-covalently adsorbed protein A for such treatment is described in Terman et al. (1981) N. Engl. J. Med. 305:1195-1200 and EPO 079,221, while the use of protein A bound to silica for such treatment is reported in Bensinger et al. (1982) N. Eng. J. Med. 306:935; Kiprov et al. (1984) J. Biol. Res. Mod. 3:341-346; Kinet et al. (1986) Eur. J. Clin, Invest. 16:43-49 and 50-55; EPO 172,018; and U.S. Patent No. 4,614,513. All treatment protocols described in the foregoing were continuous procedures usually involving treatment of at least 3 liters of plasma.

MacKintosh et al. (1983) West. J. Med. 139:36-40, discloses the treatment of cancer patients by perfusing small volumes of patient plasma over protein A bound to an agarose gel and returning the plasma to the patient in a non-continuous procedure. Some tumor regression was observed. Such agarose gel columns, however, are not inert and can release substances into the plasma. MacKintosh et al. observed toxic reactions including hypercalcemia, broncospasms, hypotension, as well as fever and chills. Moreover, even when several toxic effects are not observed, use of such columns over time may result in hypersensitization of the patient to the released substances, preventing further treatment with the agarose gel column. Jones et al. (1984) J. Biol. Resp. Mod. 3:286-292, discloses perfusion of small volumes of plasma from cats suffering from lymphosarcoma and feline leukemia virus infection. Clearing of viremia and tumor regression were observed in a number of the cats.

## SUMMARY OF THE INVENTION

According to the present invention, an improved method for removing immunoglobulin-G and immune complexes from patient plasma provides effective therapy having reduced side effects when compared to prior treatment modalities. The method comprises (1) removing a small volume of blood, typically in the range from 100 to 600 ml, more usually in the range from 400 to 500 ml, (2) separating the entire volume of blood into cellular components and plasma, (3) returning the cellular components substantially immediately to the patient, (4) contacting the plasma with an immunoadsorbent composed of protein A covalently bound to an inert, non-colloidal support matrix, typically amorphous silica, and (5) returning the plasma to the patient, usually within 15 to 30 minutes of removal, although a longer time is acceptable.

The treatment protocol just described has been found to substantially decrease the incidence of nausea, chills, fever, vomiting, and other relatively minor symptoms associated with prior treatment modalities. More importantly, more severe symptoms, such as hypotension, hypertension, and congestive heart failure, are significantly reduced when the method of the present invention is employed. Such reduced side effects are achieved, however, without any significant loss of therapeutic effectiveness.

## BRIEF DESCRIPTION OF THE DRAWINGS

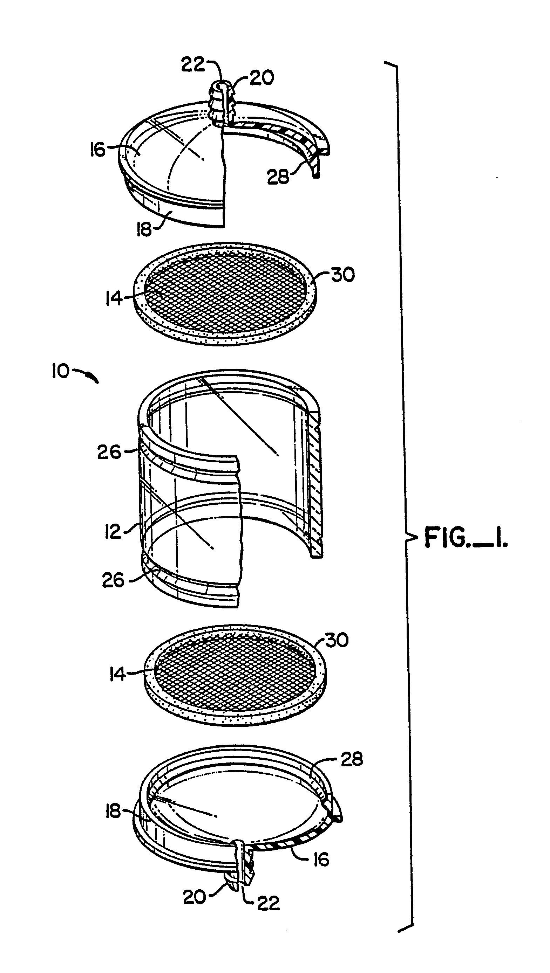

Fig. 1 is an exploded view of an immunoadsorbent column suitable for use in the method of the present invention.

## DESCRIPTION OF THE SPECIFIC EMBODIMENTS

A method for the extracorporeal removal of immunoglobulin-G and its immune complexes involves the removal of small, discrete volumes of blood from a patient, separation of the blood into its cellular and plasma components, substantially immediate reinfusion of the cellular components to the patient, removal of the IgG and immune complexes from the plasma by passage of the plasma over a protein A column, and reinfusion of the treated plasma to the patient. By treating only small volumes of blood, usually less than 600 ml, and treating the plasma with an inert, non-colloidal matrix covalently bound to protein A, the side effects of such treatment have been significantly reduced without any significant reduction in the therapeutic effectiveness. No severe, life threatening side effects, such as hypotension, hypertension, and congestive heart failure, have been observed with this procedure, and those side effects which are observed are transient and clinically manageable, being of the type normally observed in routine dialysis and therapeutic apheresis procedures.

The method of the present invention is suitable for treatment of a variety of neoplastic and autoimmune diseases. Neoplastic diseases, i.e., cancers, which may be treated include those in which an increased level of immune complexes has been observed in the sera of patients suffering from the disease. Such neoplastic diseases include melanoma, breast carcinoma, ovarian carcinoma, gastric carcinoma, colon carcinoma, lung carcinoma, testicular carcinoma, various sarcomas, including Kaposi's sarcoma and osteosarcoma, hepatoma, neuroblastoma, carcinoid tumors, acute and chronic leukemias, and Hodgkin's disease. Autoimmune diseases suitable for treatment by the method of the present invention include those having pathologic levels of IgG and immune complexes resulting from an unregulated immune response. Such autoimmune diseases include rheumatoid arthritis, systemic lupus erythematosus, myasthenia gravis, glumerulonephritis, immune thrombocytopenic purpura, vasculitis, and the like.

An immunoadsorbent material suitable for use in the present invention comprises protein A covalently coupled to an inert non-colloidal matrix under particular conditions which have been found to maximize activity of the protein A and binding capacity of the column while minimizing leakage of the protein A and other substances from the column during use.

Protein A is a cell surface protein which is isolated from particular strains of Staphylococcus aureus and is able to bind free IgG and IgG-complexes. IgG-complexes are antigen-IgG complexes which can circulate in plasma and/or other bodily fluids and are not removed by the normal phagocytic mechanisms of the immune system. The immunoadsorbent material of the present invention will have a binding capacity of at least 5 mg IgG/gm adsorbent, usually being about 6 mg/gm or greater.

Protein A may be obtained from cultures of Staphylococcus aureus for example S. aureus Cowan I, by harvesting the bacterial cells and lysing with a suitable lytic agent, such as lysostaphin, or by isolating secreted protein A directly from the bacterial growth media. The protein A may then be purified by any suitable technique, such as ion exchange combined with molecular sieve chromatography, to a final purity of 90-99%, usually 95% or greater. Alternatively, suitably purified protein A may be obtained from a number of commercial suppliers, such as IMRE Corporation, Seattle, Washington.

The inert, non-colloidal matrix will usually be a particulate or small bead, such as particulate silica, glass beads, glass, or the like. Preferred are non-colloidal particulate silicas including microcrystalline silicas, such as diatomites; crystalline silicas, such as quartz; and organic polymers of suitable inertness and chemical characteristics. The silica or other inert particulate bead should have a particle size in the range from about 30 to 120 mesh, usually in the range from 45 to 60 mesh.

In the preferred embodiment, the solid-phase matrix of the immunoadsorbent material will be formed from non-polymeric diatomite aggregates. Usually, the diatomite material will be calcined to remove any remaining organic material and to harden the surface of the aggregates in order to lessen breakage and degradation of the immunoadsorbent during use. The diatomite material will consist of silica (silicon dioxide) with lesser amounts of other minerals, including aluminum oxide, calcium oxide, magnesium oxide, ferric oxide, and the like. Usually, the diatomite material will comprise at least 80% silica, with less than 5% by weight of any other mineral. Other impurities may be present in the diatomite, but care should be taken that such impurities are non-toxic and non-degradative to the biological fluid being treated. A particularly suitable solid-phase silica (diatomite) matrix may be obtained from Johns-Manville Corporation under the tradename Chromosorb®.

The protein A is covalently coupled to the solid-phase silica matrix by derivatizing the matrix to introduce reactive functional groups, and reacting the derivatized matrix with a coupling agent or under chemical conditions which bind the protein A to the matrix. Exemplary protocols for such binding are as follows.

Amino groups may be introduced to the silica matrix as the reactive functional group by any suitable method. For example, the silica matrix is first acid washed, followed by extensive rinsing with water and drying. The acid washed silica is then reacted in a 5% to 10% solution of an aminosilane, such as $\gamma$-aminopropyltriethoxysilane, with the pH adjusted to about 3-4. After 2 hours at about 75°C, the silica matrix is again washed extensively with water and dried overnight at 100°C.

Carboxyl groups may be introduced to the silica matrix as the reactive functional group by further reacting the amino-derivatized material, as just described, with succinic anhydride as follows. The silica matrix is mixed with succinic anhydride in a suitable buffer, such as 0.5 M phosphate buffer, and the pH adjusted to about 6.0. After 12 to 16 hours at room temperature, the silica matrix is extensively washed, and dried.

Hydroxyl groups (in addition to those hydroxyl groups occurring in the native structure of the matrix) may be introduced to the silica matrix by any suitable method. For example, the silica matrix is first acid washed, rinsed extensively with water, and dried. The acid washed silica is then reacted in a 5-10% solution of a silane such as $\gamma$-glycidoxypropyltrimethoxysilane. After a 2 hour incubation at 75°C, the silica matrix is again washed extensively with water and dried at 100°C.

Once the silica matrix has been derivatized with either amino and/or carboxyl groups, the protein A is introduced by reaction with a carbodiimide which forms a covalent link between the matrix and the protein A. The carbodiimide will have the formula:

$$R'\text{-}N = C = N\text{-}R''$$

where R' and R'' may be the same or different, being either alkyl, substituted-alkyl, benzyl, substituted-benzyl, or hydrogen. Alkyl or substituted-alkyl may be straight, branched or cyclic, and R will usually have fewer than 16 atoms other than hydrogen, more usually fewer than 12 atoms, and six or fewer heteroatoms (i.e., other than carbon and hydrogen). If substituted-benzyl, R will usually have three or fewer substitutions which will typically be halogen atoms. Suitable carbodiimides are well known in the art. The preferred carbodiimide is 1-cyclohexyl-3-(2-morpholinoethyl)-carbodiimide metho-p-toluenesulfonate.

The binding reaction for the amino-derivatized matrix is carried out under the following conditions. The protein A is mixed in water in the presence of the carbodiimide. The pH of the solution is adjusted to the range from 3.5 to 4.5, usually about 3.85, and the silica matrix is introduced and gently mixed for an extended period, usually about 1 to 24 hours, more usually about 20 to 24 hours at room temperature. The matrix is then extensively washed with water, dried, and acid washed at a pH from about 2.0 to 3.0, usually about 2.25, to remove labile protein and other substances which are non-covalently bound to the silica matrix. The material is then finally washed, dried and checked for the presence of pyrogens. A suitable test for the presence of pyrogens is the limulus amoebocyte lysate (LAL) test, commercially available as a kit from Marine Biologicals, Inc., P. O. Box 546, Marmora, New Jersey 08222.

The binding process for the carboxyl-derivatized silica matrix is as follows. A carbodiimide (as above) is dissolved in water, and the solution is adjusted to a pH in the range from 3.5 to 4.5, usually about 3.85 pH. After introducing the silica matrix, the solution is gently mixed for an extended period, usually about 1 to 24 hours, more usually about 12 to 20 hours, at room temperature. The silica matrix is then removed and extensively washed with water. The protein A is then dissolved in water, the pH adjusted to the range from 3.5 to 4.5, usually about 3.85, and the silica matrix added and mixed for about 1 to 24 hours, usually about 20 to 24 hours at room temperature. The silica matrix is then extensively washed with water, dried, and washed one time in an acid wash (pH 2.0 to 3.0, usually about 2.25) to remove non-covalently bound protein A and other substances. The silica matrix is then washed a final time, and checked for pyrogens.

The binding process for the hydroxyl derivatized silica matrix is as follows. Cyanogen bromide is dissolved in water. The silica matrix is added to water and the pH is adjusted to 11.0. The cyanogen bromide solution is added to the silica matrix, the mixture is constantly stirred keeping the silica particles in suspension, and the pH is maintained between 11.0 and 11.5 by addition of NaOH until pH stabilization occurs. The activated silica matrix is extensively washed with water, mixed with a solution of protein A with the pH adjusted to 8.5 - 9.0, and mixed overnight at 25°C. After coupling, the matrix is washed extensively with water, dried, and washed one time in an acid wash, pH 2.0 - 3.0, to remove non-covalently bound and acid labile protein A linkages. The silica matrix is washed a final time and checked for pyrogens.

An exemplary protocol for preparing a microcrystalline silica-protein A immunoadsorbent suitable for use in the present invention is as follows. Acid washed silica matrix (Chromosorb® P, No. C5889, Johns-Manville, 1.25 kilograms) is weighed out, divided into 4 parts, and added to four Fernback type flasks. The matrix is re-hydrated with water and vigorously shaken overnight in a gyrotary shaker at approximately 150 rpm. After this procedure, the silica matrix is extensively washed with water to remove generated fine particles. This procedure appears to make the shape of the silica matrix particles more uniform resulting in matrix particles which generate few fines in later handling procedures. After washing, the silica matrix is added to an approximately 5-10% solution of α-glycidoxypropyltri methoxysilane, incubated for 2 hours at 75°C, extensively washed with water, and baked dry at 115°C.

The dried silanized silica matrix (1 kilogram) is re-hydrated and extensively washed with water to remove generated fines. The silica matrix is then mixed with 2 grams of protein A and 50 grams of carbodiimide (1-cyclohexyl-3-(2-morpholinoethyl) carbodiimide metho-p-toluenesulfonate) and the pH of the mixture adjusted to 3.85.

The mixture is gently rotated on a roller apparatus for 22 hours at 25°C. The silica matrix is then extensively washed with water, dried at 37°C, and the uptake of protein A is determined. After drying, 2 liters of acid water, pH 2.0 - 3.0, are added to the silica matrix, and the mixture incubated for 5 minutes at 25°C. The matrix is extensively washed with water, dried, and may be stored or used immediately.

Referring now to Fig. 1, the construction of a suitable cartridge 10 for containing the immunoadsorbent material as just described is illustrated. The cartridge comprises a cylinder 12, a pair of retaining screens 14, and a pair of end caps 16. The end caps 16 each include a flange element 18 projecting from one surface thereof and a connector nipple 20 projecting from the other surface thereof. The connector nipple includes an axial passage 22 therethrough to define inlet/outlet ports through the end caps 16.

The cylinder 12 includes an annular groove 26 at each end thereof. The flange element 18 on each end cap includes a mating ring 28 on the inner cylindrical surface thereof, which mating ring engages the annular groove 26 when the caps are placed over the end of the cylinder 12. Each screen 14 includes a gasket 30 around its circumference, which gasket serves as a sealing member between the end cap 16 and the cylinder 12 when the cartridge 10 is assembled. To assemble the cartridge 10, a first screen 14 is placed over one end of the cylinder 12, and an end cap 16 is fitted over the screen 14. The cylinder 12 is then filled with the immunoadsorbent material as described above, and assembly of the cartridge completed by placing the remaining screen 14 and end cap 16 in place.

The dimensions of the cartridge 10 are not critical, and will depend on the desired volume of immunoadsorbent material. The volume of the cylinder 12 will typically range from about 50 to 500 cc, having a diameter in the range from about 4 to 8 cm and a length in the range from about 5 to 10 cm. The columns may be sterilized, typically with a gas sterilant such as ethylene oxide, and either used immediately or sealed and stored for later use. Prior to use, the column 10 will be washed with normal saline followed by a wash with normal saline containing heparin or other suitable anticoagulant such as anti-coagulant citrate dextrose (ACD).

The method of the present invention is as follows. Blood is withdrawn from the patient by conventional venous phlebotomy to a volume from about 100 to 600 ml, usually from about 400 to 500 ml, more usually being about 500 ml. The withdrawn blood is immediately separated into its cellular components and plasma, typically by centrifugation at about 800 to 1100 rpm for a period in the range from about 5 to 20 minutes. Alternatively, a semipermeable membrane, or a conventional apheresis centrifugal machine, can be used for such separation.

After separation, the cellular components of the blood are immediately reinfused into the patient by normal procedures, such as venous infusion. The plasma is applied to the immunoadsorbent column, as described above, at a flow rate in the range from about 10 to 30 ml/min. Normal precautions should be taken to assure that the plasma flows evenly through the column so that all portions of the plasma are exposed to the immobilized protein A.

After the entire volume has passed through the column, the plasma is returned to the patient by conventional procedures, such as venous infusion or other circulatory access.

The above-described treatment protocol will be repeated periodically, usually at least every two weeks, more usually at least each week, and frequently as often as two or three times per week, and may be performed daily. A course of treatment will usually run at least four weeks, often being at least eight weeks, and can be twelve weeks or longer, or until the diseased state has responded to the therapy.

The following experiments are offered by way of illustration, not by way of limitation.

## EXPERIMENTAL

### Materials and Methods

Protein A-silica immunoadsorbent was prepared by the exemplary method described hereinbefore. The immunoadsorbent had approximately 2.0 mg of protein A for each gram of silica. The immunoadsorbent was packed into columns having 25 and 100 grams of immunoadsorbent, respectively. Columns were prepared for use by washing with 3.5 $l$ of normal saline followed by washing with 0.5 $l$ normal saline containing 5000 units heparin.

Patients were treated by either the non-continuous protocol of the present invention, or by a continuous protocol analogous to earlier methods.

For the non-continuous protocol, 500 ml of blood was removed from the patient by phlebotomy. The blood was separated into cellular components and plasma by centrifugation, and the cellular components immediately reinfused into the patient. Plasma (250 ml) was then perfused through the column (either 25 or 100 gram immunoadsorbent matrix), and the treatments repeated three times per week over a four week period. Plasma flow through the column was 10 to 20 ml/min.

For the continuous procedure, the patient was given an anticoagulant, either heparin or ACD, and blood was withdrawn continuously through a venous access line. The whole blood was separated into cellular and plasma components using commercially available cell separators, and the blood withdrawn at a rate sufficient to provide a plasma flow of 10 to 20 ml/min. The plasma return line from the cell separator was connected to the protein A column so that the plasma flowed upward through the column. The treated plasma and cellular components were reinfused simultaneously into the patient, and treatment terminated after a plasma volume of either 250 ml or 2 liters was completed. Termination was accomplished by normal saline displacement until the blood tubing was clear.

## Results

### Cancer

Cancer patients entered into the studies were randomly assigned to one of eight treatment regimens defined by three variables: (1) the amount of protein A on an immunoadsorbent column (50 or 200 mg), (2) the treatment schedule (three treatments per week for four weeks or one treatment per week for twelve weeks), and (3) the treatment mode (continuous procedure utilizing an immunoadsorbent column in line continuously with an apheresis machine for treatment of 2 liters of plasma, or a discontinuous procedure without use of an apheresis machine for perfusion of 250 ml of plasma). Each continuous treatment involved separation of plasma from whole blood formed elements, passage of plasma through the immunoadsorbent column, recombination of treated plasma with the blood cells, and reinfusion of the mixture into the patient.

Each discontinuous treatment involved collection of blood by phlebotomy, centrifugation of the blood, collection of the plasma, and immediate return of the cellular elements to the patient. The plasma was passed through an immunoadsorbent and subsequently returned to the same patient by intravenous infusion.

One thousand ninety-four treatments with immunoadsorbent columns were evaluated for side effects. A variety of side effects were observed, the majority being chills, fever, and tumor pain. All side effects were transient and manageable. There were statistically significantly fewer side effects using the discontinuous treatment method as compared to the continuous method ($P \leq 0.004$).

Antitumor responses were observed among various tumor types in patients treated with immunoadsorbent columns. There were 56 patients treated with the discontinuous method and 9 had a measurable antitumor response. There were 29 patients treated with the continuous method and 13 had a measurable antitumor response. There was not a statistically significant difference between the two groups, thus the discontinuous method of treatment is as effective as the continuous method.

Autoimmune Disease

Clinical studies were conducted to investigate the safety and efficacy of immunoadsorbent columns in the treatment of the autoimmune disease immune thrombocytopenic purpura (ITP). These patients have decreased platelets due to their disease. Patients were treated using the discontinuous method with 250 ml of plasma being treated per procedure. Two hundred fourteen treatments were evaluated for side effects. The side effects observed most often were pain (associated with 29% of treatments), fever (with 32% of treatments), chills (with 23% of treatments), and rash (with 14% of treatments).

Of the 24 ITP patients treated in the clinical studies, 14 showed clinical improvement by significant increase in their platelets. Thus, 58 percent of these treated patients responded to discontinuous treatment. Responding patients also had measurable and significant decreases in their antibody levels to platelets. The responding patients also had significant decreases in their levels of immune complexes as mesured by the C1q-immune complex assay.

Although the foregoing invention has been described in some detail by way of illustration and example for purposes of clarity of understanding, it will be obvious that certain changes and modifications may be practiced.

**Claims**

1. A method for treating a patient to modulate the amount of immunoglobulin G and immune complexes in the patient's blood, said method comprising:

removing a preselected volume of blood from the patient;

separating the blood volume into cellular components and plasma;

reinfusing the cellular components of the blood to the patient substantially immediately;

contacting the plasma with an immunoadsorbent comprising protein A covalently attached to an inert, non-colloidal support; and

returning the plasma to the patient a preselected time period after reinfusion of the cellular blood components has been completed.

2. A method as in claim 1, wherein the preselected volume of blood is in the range from about 100 to 600 ml.

3. A method as in claim 1, wherein the blood is separated by centrifugation.

4. A method as in claim 1, wherein the blood is separated by passage through a semi-permeable membrane.

5. A method as in claim 1, wherein the blood is separated by an apheresis centrifugal machine.

6. A method as in claim 1, wherein the cellular components are reinfused to the patient within 15 to 30 minutes of blood removal.

7. A method as in claim 1, wherein the immunoadsorbent comprises protein A covalently attached to a microcrystalline silica matrix.

8. A method as in claim 7, wherein from about 1 to 5 mg of protein A are attached to each gram of microcrystalline silica.

9. A method as in claim 7, wherein the immunoadsorbent is prepared by:

introducing free amino or carboxyl groups onto the silica matrix:

reacting the silica matrix with purified protein A in the presence of a carbodiimide at a pH in the range from 3.5 to 4.5 to covalently link the protein A to the matrix through the amino or carboxyl groups; and

washing the silica matrix at a pH in the range from 2.0 to 3.0 to remove loosely bound protein A from the matrix.

10. A method as in claim 9, wherein the microcrystalline silica matrix is diatomite.

22 20

16

28

18

30

14

10

26

12

26

30

14

28

18

16

20 22

# FIG._I.